# EUROPEAN PATENT SPECIFICATION

(11) **EP 2 011 573 B1**
(45) Date of publication and mention of the grant of the patent: **21.07.2010**
(21) Application number: 07119995.4
(22) Date of filing: 05.11.2007
(51) Int. Cl.: B01L 3/00

(54) **Freezing a microfluidic chip**
Einfrierung eines mikrofluidischen Chips
Congélation d'une puce microfluidique

(43) Date of publication of application: 07.01.2009
(73) Proprietor: Agilent Technologies, Inc., Santa Clara CA 95051 (US)
(72) Inventor: Ruefer, Andreas, 76137, Karlsruhe (DE)
(74) Representative: Barth, Daniel Mathias

(56) References cited:
- WO-A-2004/074818
- WO-A-2005/075971
- WO-A-2005/093389
- US-A1- 2002 160 361
- US-A1- 2006 094 108

## Description

### BACKGROUND ART

The present invention relates to microfluidic chips comprising a micro-channel system.

In microstructure technology applications, as in the Agilent 2100 Bioanalyzer by the applicant Agilent Technologies, fluid may be conveyed through miniaturized channels (which may be filled with gel material) formed in a substrate. For a capillary electrophoresis device, as an example for such a microstructure technology application, an electric field is generated in the fluid channels in order to allow for a transport of components of the fluid through the channels using electric forces. Such an electric force or field may be generated by dipping contact pins of the capillary electrophoresis device into the fluid which may be filled in a well defined by a carrier element coupled to a microfluidic chip, and by applying an electrical voltage to such contact pins.

WO 2005/075971 A1, WO 00/78454 A1, DE 19928412 A1, and US 6,814,846 by the same applicant Agilent Technologies show different microfluidic chips and applications. Other microfluidic devices and applications are disclosed e.g. in WO 98/49548, US 6,280,589, or WO 96/04547.

WO 2005/093389 A1 discloses a method for preparing a sample for matrix-assisted laser desorption/ionization (MALDI). The sample is provided into a microfluidic structure, which is then frozen, lyophilized, and eventually applied for MALDI.

### DISCLOSURE

It is an object of the invention to provide an improved handling of microfluidic chips. The object is solved by the independent claim. Further embodiments are shown by the dependent claim.

Embodiments of the present invention relate to a method for preparing a microfluidic chip, with the microfluidic chip comprising a micro-channel system with at least one micro-channel. The method comprises supplying one or more fluids to at least one predefined section of the micro-channel system or to the whole micro-channel system, and freezing the entire microfluidic chip by exposing the microfluidic chip to a temperature below -5°C, wherein the one or more fluids contained in the chip to a temperature below -5°C, wherein the one or more fluids contained in the micro-channel system are frozen solid.

According to embodiments of the present invention, after various fluids have been supplied to predefined sections of the chip, the chip is frozen. The fluids may e.g. comprise buffers, reference samples, different kinds of solutions or solvents, etc. The fluids contained in the chip's micro-channel system are frozen solid. Now, the chip is transported. During transport, the frozen fluids are retained in the chip, whereby leakage of the fluids is avoided.

In the frozen state, the microfluidic chip, which comprises one or more frozen fluids, is transported to any desired location. There, the chip may be stored in a freezer. For using the chip, the chip is thawed. The unfrozen chip is ready to be used. It has been verified experimentally for a multitude of chips that freezing and thawing does not significantly affect the proper functioning of the chip.

In prior art solutions, it has been impossible to transport a microfluidic chip to which a variety of different fluids have been supplied. Therefore, it has been necessary to perform chip priming at the location where the chip is actually utilized.

According to embodiments of the present invention, chip priming does not have to be performed by the user any longer. Instead, buffers and other fluids may be provided to the chip at the manufacturer's site. Then, the chip is frozen and shipped to the customer. Hence, the burden of supplying various fluids to the chip may be transferred from the user to the manufacturer. The customer does not have to care about chip priming any more. Furthermore, the manufacturer may have a more suitable equipment and the necessary skills for loading the fluids to the chip.

However, the method according to embodiments of the present invention is not limited to chip priming. The method may be used whenever microfluidic chips have to be transported between different locations. For example, it may be desirable to analyze samples from different locations at one central laboratory. In this case, each of the samples may be provided to a respective microfluidic chip, which is frozen and sent to the central laboratory. There, samples from different locations may be analyzed.

According to a preferred embodiment, the method comprises exposing the microfluidic chip to a temperature between -5 °C and -80 °C, further preferably between -15°C and -25°C. This temperature range is most appropriate for freezing the microfluidic chip that is filled with various different solutions, reagents, etc. In this temperature range, relaxation of the stress and strain that occurs during freezing is possible. Furthermore, by choosing a freezing temperature below -5°C, it is made sure that even fluids of a certain ionic strength are frozen solid in a reliable manner.

According to a further preferred embodiment, the microfluidic chip comprises a sample separation system adapted for separating compounds of a given sample. It is no longer necessary to perform chip priming before separation of the given sample can be started. According to embodiments of the present invention, the user simply has to thaw a microfluidic chip that comprises all the required fluids and supply a sample to the chip. Then, a chip run may be started. The procedure for analyzing a sample is simplified. Furthermore, the time required for analyzing a sample is reduced.

According to a preferred embodiment, the microfluidic chip is an electrophoresis chip adapted for electrophoretically separating compounds of a given sample. In an electrophoresis chip, an electric field is applied across a separation device comprising a sieving medium, and the sample is passed through the separation device. During their passage through the separation device, the sample's different compounds are separated in accordance with their respective mobilities. It has been found that freezing and thawing of the electrophoresis chip does not impair the chip's functionality. In particular, the freezing and unfreezing operations do not seem to have a significant impact on the respective sieving medium.

According to a preferred embodiment, the microfluidic chip is realized as a multilayer structure. Preferably, the multilayer structure comprises at least two thermally bonded glass sheets or plastic sheets. Preferably, the microfluidic chip further comprises a plastic caddy with a plurality of fluid reservoirs for supplying different fluids to the multilayer structure. Further preferably, the microfluidic chip comprises a plastic caddy attached to the multilayer structure with an adhesive.

According to a preferred embodiment, material properties of the plastic caddy and the adhesive are such that the plastic caddy does not detach when being cooled down to a temperature below -5°C. For example, the coefficient of thermal expansion of the plastic caddy and of the chip material may be similar to one another. Thus, it is made sure that thermal stress and strain that occurs during the freezing operation does not cause a detachment of the plastic caddy.

In a preferred embodiment, the fluids supplied to the micro-channel system comprise one or more of a background buffer, a background buffer including a gel matrix, a sample buffer, a reference sample buffer, a destaining solution, a dye solution. One or more of these fluids may be supplied to the microfluidic chip before it is frozen. For example, a manufacturer may supply fluids required for the chip's operation to the chip before freezing the chip.

Preferably, the method further comprises supplying a background buffer including a gel matrix to at least one predefined section of the micro-channel system or to the whole micro-channel system. When the background buffer with the polymer chains dissolved therein is exposed to a freezing operation, no phase segregation of buffer and polymer is observed. It has been verified experimentally that the polymers do not tend to aggregate. Hence, both the structure and the functionality of the gel matrix are preserved during the freezing operation.

According to a preferred embodiment, the background buffer comprises a gel matrix with a certain concentration of polymer chains. Preferably, the gel matrix comprises polydimethylacrylamide, also referred to as PDMA. Preferably, the microfluidic chip comprises an electrophoretic separation channel for performing an electrophoretic separation of sample compounds passing through the separation channel. In a preferred embodiment, a background buffer including a gel matrix is supplied to the separation channel. Preferably, the gel matrix is adapted for sieving the sample's compounds passing through the separation channel.

Further preferably, an overpressure between 0.1 and 20 bar is applied for supplying background buffer and gel matrix to at least one predefined section of the micro-channel system or to the whole micro-channel system. The viscosity of the background buffer with the gel matrix is rather high, and therefore, an overpressure between 0.1 and 20 bar is helpful for supplying background buffer and gel matrix to defined sections of the micro-channel system. For example, the background buffer with the polymers dissolved therein may be supplied to the separation channel of the microfluidic chip. According to embodiments of the present invention, this loading procedure does not have to be performed by the user any longer. Instead, background buffer and gel matrix may be provided to the chip at the manufacturer's site. Then, the chip is frozen and shipped to the customer. Hence, the burden of supplying background buffer and gel matrix to the chip is transferred from the user to the manufacturer.

Preferably, in case the chip is prepared for use in protein analysis, a background buffer comprising Tris, Tricine and SDS is employed. SDS is an agent that may e.g. be used for denaturing and solubilizing protein species.

Preferably, in case the chip is prepared for analysing at least one of RNA and DNA fragments, a background buffer comprising Tris(hydroxymethyl)methyl-aminopropane sulfonic acid, also referred to as TAPS, is employed.

In a preferred embodiment, the microfluidic chip comprises a plurality of wells fluidically coupled with the micro-channel system. Further preferably, the method further comprises supplying a background buffer to one or more of the wells.

According to an alternative embodiment, the microfluidic chip may not comprise any wells at all. Instead of supplying fluids to respective wells, the fluids may be directly supplied to inlets of the chip's micro-channels.

According to a preferred embodiment, the method comprises supplying a reference sample comprising a set of well-defined moieties to one or more of: at least one dedicated micro-channel of the micro-channel system, and at least one dedicated well fluidically coupled with the micro-channel system. The reference sample may e.g. comprise a set of well-defined species, e.g. protein species, RNA species or DNA species. The reference sample can be used for calibrating acquired spectra. For example, the species contained in the reference sample may correspond to well-known peaks in the acquired spectra, and by identifying these reference peaks, the acquired spectra may be calibrated.

According to a preferred embodiment, the method comprises supplying a sample buffer to one or more of: at least one dedicated micro-channel of the micro-channel system, and at least one dedicated well fluidically coupled with the micro-channel system. Preferably, the ionic strength of the sample buffer is considerably lower than the ionic strength of the background buffer. As a consequence, when sample ions pass from the low-conductivity sample buffer to the high-conductivity background buffer, an increase of the concentration of sample ions is observed. This effect is referred to as "stacking". By taking advantage of this effect, the signal-to-noise ratio of acquired spectra may be improved.

Preferably, the sample buffer comprises at least one of: Tris, Tricine, TAPS, a lower marker species, an upper marker species. For example, the lower marker species and the upper marker species may be used for aligning different spectra.

According to a preferred embodiment, the method comprises supplying a destaining solution to one or more of: at least one dedicated micro-channel of the micro-channel system, and at least one dedicated well fluidically coupled with the micro-channel system. In case background fluorescence of a sample solution is too high, a destaining solution may be supplied to the sample solution, in order to dilute the sample solution and reduce background fluorescence. This process is commonly referred to as "destaining". By reducing the amount of background fluorescence, detection sensitivity may be increased.

According to a preferred embodiment, the method comprises supplying a dye solution to one or more of: at least one dedicated micro-channel of the micro-channel system, and at least one dedicated well fluidically coupled with the micro-channel system. The dye solution may e.g. be supplied to a detection flow path, in order to simplify adjustment of an external detection unit's focus.

Preferably, freezing conditions are chosen such that phase separation of background buffer and gel matrix is avoided. Further preferably, freezing conditions are chosen such that mechanical damage to the microfluidic chip is avoided. In particular, it is important to allow for a relaxation of thermal stress and strain that builds up during the freezing process.

In a preferred embodiment, the method comprises protecting the microfluidic chip with a foil or a cover, in order to prevent sublimation of the frozen fluids. In this way, the microfluidic chips may be stored for a certain period of time, whereby the volumes and concentrations of fluids that have been supplied to the chip remain substantially unaltered.

Preferably, preparation of the microfluidic chip is carried out at a manufacturer's site. At the manufacturer's site, chip priming is performed. Then, the microfluidic chips are frozen and shipped to the customers. A customer may unfreeze one of the microfluidic chips whenever a sample analysis is to be performed. After thawing, the chip is ready-to-use. Further preferably, preparation of the microfluidic chip is carried out at an automated manufacturing facility. Thus, chip priming may be automated and carried out at low cost.

A method for analysing compounds of a given sample according to embodiments of the present invention comprises thawing a microfluidic chip adapted to perform a sample separation, the microfluidic chip being prepared according to the above described method, supplying the sample to a sample well or to a dedicated micro-channel of the microfluidic chip, and separating and analyzing compounds of the sample. Accordingly, a user who wants to perform a sample analysis no longer has to carry out a chip priming procedure.

### BRIEF DESCRIPTION OF DRAWINGS

Other objects and many of the attendant advantages of embodiments of the present invention will be readily appreciated and become better understood by reference to the following more detailed description of embodiments in connection with the accompanied drawing(s). Features that are substantially or functionally equal or similar will be referred to by the same reference sign(s).

Fig. 1 shows the process of freezing and unfreezing a microfluidic chip; and

Fig. 2 gives a detailed view of a gel electrophoresis chip.

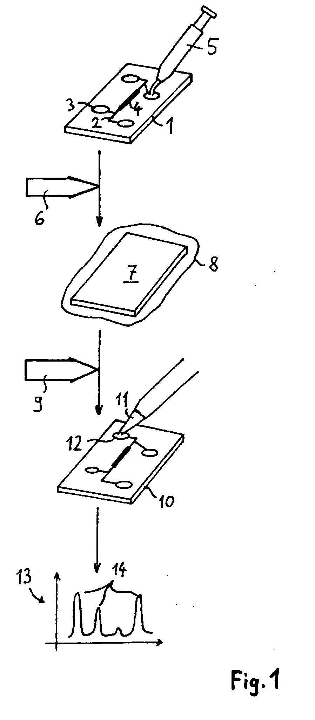

Fig. 1 illustrates a first embodiment of a method for preparing a microfluidic chip. The microfluidic chip 1 comprises a plurality of micro-channels 2. Various different fluids may be supplied directly to the micro-channels 2, or to dedicated wells 3 that are fluidically coupled with the micro-channels 2. The microfluidic chip 1 may e.g. comprise a separation device 4, e.g. a separation column, that is adapted for separating and analyzing compounds of a given sample. Separation techniques like e.g. gel electrophoresis or liquid chromatography may be employed. However, the present invention may be employed for other types of microfluidic chips as well.

Before operation of the microfluidic chip can be started, a plurality of different fluids has to be provided to the micro-channels 2 and the wells 3 of the microfluidic chip. For example, in case of a chip that is used for sample separation, one or more of the following fluids have to be supplied to the chip: a background buffer, a sample buffer, a reference sample, a dye solution, a destaining solution, etc. As shown in Fig. 1, a syringe 5 may be used for forcing the various fluids into the micro-channels 2, preferably by applying an overpressure between 0.1 and 20 bar.

In a preferred embodiment, the background buffer contains a certain concentration of polymer chains, the so-called "gel matrix". Together with the background buffer, the gel matrix is supplied to the separation device 4 and serves as a sieving medium. The syringe 5 may be used for supplying background buffer and gel matrix to the chip, whereby an overpressure of 0.1 to 20 bar is sufficient to force the polymer chains into the separation channel. Alternatively, capillary forces may be used for filling the chip with fluids.

According to a preferred embodiment, chip priming is no longer performed by the customer. Instead, the various fluids are supplied to the chip at the manufacturer's site. In step 6, after chip priming has been performed, the microfluidic chip 1 with the various fluids contained therein is frozen. The chip is brought to a temperature below 0°C, and preferably to a temperature between -20°C and -5°C. For freezing the chip and the fluids contained therein, the entire chip may be placed in a freezer until the fluids contained in the micro-channel system are frozen solid. It has to be taken into account that, due to the buffers' respective ionic strengths, the freezing points of the various buffers may be well below 0°C. In a preferred embodiment, the chip's temperature as a function of time is optimized with respect to one or more of: material properties of the chip, relaxation of mechanical stress, prevention of phase separation. This may be achieved by choosing adequate freezing conditions. For example, freezing must not be effected too quickly, in order to avoid mechanical damage to the microfluidic system.

One important aspect of the present invention is that the freezing operation does not seem to impair the structure of a background buffer with a gel matrix. Phase segregation of the polymers on the one hand and the background buffer on the other hand has not been observed. In particular, aggregation of the polymer chains during the freezing operation does not seem to occur. Therefore, both the structure and the functionality of the gel matrix in the separation channel are preserved during a freezing operation and a subsequent unfreezing operation.

Next, the frozen chip 7 is shipped to the customers. For avoiding sublimation of the frozen fluids, the frozen chip 7 may be wrapped in a plastic foil 8 during transport, or a cover may be placed on the frozen chip 7. Another strategy for avoiding sublimation of the frozen fluids is to create an atmosphere of saturated water vapour. For example, a blister package may be used for transporting the frozen chips. Furthermore, the packaging materials may comprise water reservoirs or sponges soaked with water.

The customer receives the frozen chips and keeps them in a freezer until they are needed. When the customer wants to perform a sample analysis, one of the frozen chips is defrosted (cf. step 9). The unfrozen chip 10 comprises the buffers and fluids required for performing a sample analysis. A sample of interest 11 is pipetted into a sample well 12 of the unfrozen chip 10. Then, the microfluidic chip 10 is placed in a sample analysis apparatus, and a chip run is started. The sample is conveyed through the separation channel, and the sample's compounds are separated. In a detection flow path fluidically connected to the separation channel's outlet, the various sample compounds are detected. As a result, a spectrum 13 with a multitude of peaks 14 may be obtained, with the peaks 14 corresponding to the sample's compounds.

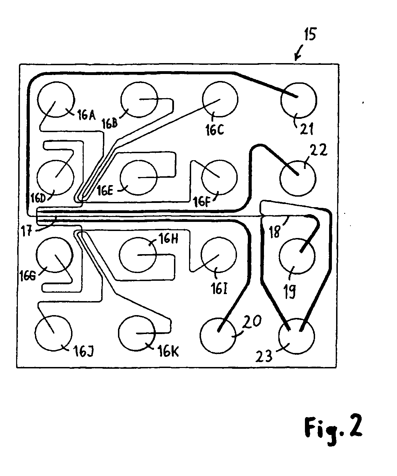

Fig. 2 depicts an electrophoresis chip 15, which is an example of a chip that may be frozen. On-chip gel electrophoresis is a powerful technique for electrophoretically separating a sample's compounds. The electrophoresis chip 15 comprises a plurality of sample wells 16A to 16K that may e.g. be filled with different samples. The sample wells 16A to 16K are fluidically connected, via respective fluid conduits, with an inlet of a gel-filled separation channel 17. A sample's charged compounds are electrokinetically moved through the separation channel 17. During their passage through the separation channel 17, the sample's compounds are separated according to their respective mobilities. In order to detect the compounds after they have been separated, the outlet of the separation channel 17 is fluidically coupled with a detection flow path 18, whereby an external detection unit may be focused onto the detection flow path 18. Via the detection flow path 18, the separation channel 17 is fluidically connected with a waste well 19. The electrophoresis chip 15 further comprises auxiliary wells 20, 21, 22 that are fluidically coupled to the inlet of the separation channel 17. One or more of the auxiliary wells 20, 21, 22 may be used for supplying a background buffer with a gel matrix to the separation channel 17, in order to fill the separation channel 17 with a sieving medium. Furthermore, the electrophoresis chip 15 comprises another auxiliary well 23 that is fluidically coupled to the inlet of the detection flow path 18.

The electrophoresis chip 15 may e.g. be implemented as a multilayer structure composed of two or more glass sheets, wherein the micro-channels are realized as grooves in a glass sheet's surface. For implementing the sample wells, a plastic caddy comprising a plurality of wells 16A to 16K and 19 to 23 may be fixed to the upper surface of the multilayer structure. Each fluid reservoir may comprise a volume of 3 to 15 µl. An UV curable acrylic based adhesive may be used for attaching the plastic caddy to the multilayer structure. In order to prevent that the plastic caddy detaches from the multilayer structure when being brought to a temperature below 0°C, the thermal expansion properties of the plastic caddy, the glass sheets and the adhesive have to match.

For separation of protein samples, a background buffer comprising at least one of Tricine and Tris is used, preferably at a pH between 7 and 8. The formula of Tris is (HO-CH₂)₃-C-NH₂ and the formula of Tricine is (HO-CH₂)₃-C-NH-CH₂-COOH. The respective concentrations of Tris and Tricine may e.g. be between 30 mM and 300 mM. Furthermore, a certain concentration of sodium or lithium dodecyl sulphate, also referred to as SDS or LiDS, may be added to the background buffer. SDS and LiDS have traditionally been used for denaturing and solubilizing protein species. A solution of SDS or LiDS comprises CH₃-(CH₂)₁₀-CH₂-O-SO₃⁻ and Na⁺ or Li⁺.

As a gel matrix, the background buffer further comprises a certain concentration of polymer chains dissolved in the background buffer. Preferably, polydimethylacrylamide (PDMA) is used as a polymer species. The background buffer with the polymers is supplied to at least one of the auxiliary wells 20, 21, 22, and by applying an overpressure between 0.1 bar and 20 bar, background buffer and polymers may be moved to the separation channel 17.

The electrophoresis chip 15 may as well be used for the separation and analysis of RNA or DNA species. However, in this case, different buffer systems are utilized. For example, for RNA and DNA separation, N-Tris(hydroxymethyl)methyl-3-aminopropane sulfonic acid, also referred to as TAPS, is commonly used as a buffer, preferably in concentrations between 30 mM and 300 mM. In order to improve the resolution of the acquired DNA or RNA spectra, a certain concentration of dimethylsulfoxide (DMSO) may be added to the buffer. The electrophoresis chip 15 may be realized as a multilayer structure that is composed of two or more glass sheets. To prevent adhesion of the RNA or DNA species to the channels' sidewalls, a certain concentration of Triton may be added to the background buffer. Also in the field of RNA and DNA analysis, PDMA polymers may be employed as a gel matrix. In addition to the separation channel 17, one or more of the sample wells 16A to 16K may also be filled with background buffer and gel matrix.

Alternatively or additionally, some of the sample wells 16A to 16K may be filled with a dedicated sample buffer. Basically, the same buffer systems that are used as a background buffer may also be employed as a sample buffer. For example, in the field of protein analysis, one or more of Tris and Tricine may be used as a sample buffer, whereas in the field of RNA or DNA separation, a buffer such as TAPS may be used as a sample buffer.

In order to take advantage of an effect referred to as "stacking", the ionic strength of the sample buffer may be considerably smaller than the ionic strength of the background buffer. When applying a voltage or a current to the respective sample well, there is a large electric field in the low conductivity sample buffer, and there is a small electric field in the high conductivity background buffer. Later, when a sample is applied to the sample well, sample ions drift within the high field sample buffer, pass through the conductivity interface region and enter the low field background buffer. As the sample ions cross the interface region between the two buffers, sample concentration increases. This increase of sample concentration, which is commonly referred to as "stacking", is highly desirable, because it gives rise to a corresponding increase in detection sensitivity.

The sample buffer supplied to one or more of the sample wells 16A to 16K may further comprise marker species, e.g. a lower marker species like aprotinin, which is a protein of low molecular weight and high mobility. Each marker species is related to one or more well-known peaks that appear in the acquired spectra, which may be used for aligning and calibrating the acquired spectra.

Chip priming may further comprise supplying fluids for special purposes to one or more dedicated wells of the electrophoresis chip 15. For example, a reference sample comprising a set of well-known species may be supplied to one of the sample wells 16A to 16K. The reference sample is used for calibrating spectra acquired with the electrophoresis system. The set of well-known species contained in the reference sample corresponds to a set of well-known reference peaks in a corresponding peak pattern. Therefore, the reference sample is often referred to as a "ladder".

Chip priming may further comprise supplying a destaining solution to an auxiliary well 23. In case fluorescence detection is used for detecting sample species passing through the detection flow path 18, the concentration of fluorescence dye in the background buffer may be quite high. In order to reduce background fluorescence, a continuous flow of destaining solution may be supplied to the sample solution after it has passed the separation channel 17, and before it reaches the detection flow path 18. Destaining is a useful strategy for improving the signal-to-noise ratio of acquired spectra.

Alternatively or additionally, a dye solution may be supplied to one of the wells on the electrophoresis chip 15. The dye solution may e.g. be used for adjusting the focus of an external fluorescence detection unit. Forthis purpose, the dye solution is electrokinetically moved to the detection flow path 18, and then, the fluorescence detection unit is focussed with respect to the detected fluorescence pattern.

After chip priming has been performed, the entire electrophoresis chip 15 is frozen. Preferably, the electrophoresis chip 15 is brought to a temperature between -80°C and -5°C, further preferably between -25°C and -15°C. Freezing of the electrophoresis chip 15 may e.g. be effected by placing the electrophoresis chip 15 in a freezer or in a freezer compartment. The freezing process may be optimized with respect to time and temperature, to avoid segregation or precipitation of the supplied species, and physical damage to the chip system. For example, freezing must not take place to quickly, in order to prevent mechanical damages of the micro-channel system. The finding of the present invention is that freezing and thawing of the microfluidic chip does not destroy the chip's functionality. It has been confirmed experimentally that the functionality of the gel matrix in the separation channel 17 is preserved during the freezing and unfreezing operation.

The frozen chips may be delivered to the customers. After defrosting one of the chips, a customers may supply respective samples of interest to the chip's sample wells and start a chip run to analyze the samples.

The idea of freezing and thawing a microfluidic chip may be used in various different applications. For example, there may arise a need for analyzing samples at a variety of different locations. At each location, chip priming of a microfluidic chip is performed, and one or more samples of interest are supplied to the microfluidic chip. Then, the respective chips are frozen. The frozen chips with the samples of interest are sent to a central analysis facility. Accordingly, in this embodiment, the sample of interest is supplied to the microfluidic chip before the chip is frozen.

At the central facility, frozen chips from a variety of different locations are received, unfrozen and analyzed with suitable equipment. Instead of providing each of the local facilities with separate analysis equipment, the hardware and software required for performing chip runs and analyzing the spectra only has to be maintained at the central facility. The equipment only has to be purchased once, which leads to a considerable reduction in cost.

## Claims

1. A method for analysing compounds of a given sample (11) by using a microfluidic chip (1) comprising a micro-channel system with at least one micro-channel (2), the method comprising
supplying one or more fluids to at least one predefined section of the micro-channel system or to the whole micro-channel system,
freezing (6) the entire microfluidic chip (1) by exposing the microfluidic chip (1) to a temperature below -5°C, wherein the one or more fluids contained in the micro-channel system are frozen solid,
transporting the microfluidic chip (7),
thawing (9) the microfluidic chip (7),
supplying the sample (11) to a sample well (12) or to a dedicated micro-channel of the microfluidic chip (7), and
separating and analyzing compounds of the sample (11).

2. The method of claim 1, wherein
the microfluidic chip comprises a sample separation system adapted for separating, preferably electrophoretically, compounds of a given sample.

3. The method of claim 1 or 2, wherein
the microfluidic chip is realized as a multilayer structure.

4. The method of claim 1 or any one of the above claims, wherein the fluids supplied to the micro-channel system comprise one or more of a background buffer, a background buffer including a gel matrix, a sample buffer, a reference sample buffer, a destaining solution, a dye solution.

5. The method of claim 1 or any one of the above claims, further comprising: supplying a background buffer including a gel matrix to at least one predefined section of the micro-channel system or to the whole micro-channel system.

6. The method of claim 1 or any one of the above claims, wherein
in case the chip is prepared for use in protein analysis, a background buffer comprising Tris, Tricine and SDS is employed.

7. The method of claim 1 or any one of the above claims, wherein
in case the chip is prepared for analysing at least one of RNA and DNA fragments, a background buffer comprising Tris(hydroxymethyl)methyl-aminopropane sulfonic acid, also referred to as TAPS, is employed.

8. The method of claim 1 or any one of the above claims, wherein the microfluidic chip comprises a plurality of wells fluidically coupled with the micro-channel system, the method further comprises supplying a background buffer to one or more of the wells.

9. The method of claim 5, wherein
freezing conditions are chosen such that phase separation of background buffer and gel matrix is avoided.

10. The method of claim 1 or any one of the above claims, wherein the method further comprises
protecting the microfluidic chip with a foil or a cover, in order to prevent sublimation of the frozen fluids.

## Patentansprüche

1. Verfahren zum Analysieren der Verbindungen einer vorgegebenen Probe (11) unter Verwendung eines Mikrofluid-Chips (1), der ein Mikrokanalsystem mit mindestens einem Mikrokanal (2) aufweist, wobei das Verfahren die folgenden Schritte aufweist:
Einführen eines oder mehrerer Fluide in mindestens einen vordefinierten Abschnitt des Mikrokanalsystems oder in das gesamte Mikrokanalsystem,
Tiefkühlen (6) des gesamten Mikrofluid-Chips (1) durch Einwirken einer Temperatur unter -5°C auf den Mikrofluid-Chip (1), wobei das eine oder die mehreren im Mikrokanalsystem enthaltenen Fluide einen tiefgekühlten Feststoff darstellen,
Transportieren des Mikrofluid-Chips (7),
Auftauen (9) des Mikrofluid-Chips (7),
Einführen der Probe (11) in eine Probenmulde (12) oder in einen speziellen Mikrokanal des Mikrofluid-Chips (7) und
Trennen und Analysieren der Verbindungen der Probe (11).

2. Verfahren nach Anspruch 1, wobei
der Mikrofluid-Chip ein Probentrennsystem aufweist, das zum Trennen der Verbindungen einer vorgegebenen Probe, vorzugsweise durch Elektrophorese, dient.

3. Verfahren nach Anspruch 1 oder 2, wobei
der Mikrofluid-Chip als Mehrschichtstruktur ausgeführt ist.

4. Verfahren nach Anspruch 1 oder einem der vorhergehenden Ansprüche, wobei die in das Mikrokanalsystem eingeführten Fluide eine oder mehrere der folgenden Komponenten aufweisen: einen Hintergrundpuffer, einen Hintergrundpuffer mit einer Gelmatrix, einen Probenpuffer, einen Referenzprobenpuffer, eine Entfärbungslösung, eine Farbstofflösung.

5. Verfahren nach Anspruch 1 oder einem der vorhergehenden Ansprüche, das ferner den folgenden Schritt aufweist: Einführen eines Hintergrundpuffers mit einer Gelmatrix in mindestens einen vordefinierten Abschnitt des Mikrokanalsystems oder in das ganze Mikrokanalsystem.

6. Vorrichtung nach Anspruch 1 oder einem beliebigen der vorhergehenden Ansprüche, wobei
ein Hintergrundpuffer verwendet wird, der Tris, Tricin und SDS aufweist, wenn der Chip zur Verwendung für die Proteinanalyse vorbereitet wird.

7. Vorrichtung nach Anspruch 1 oder einem beliebigen der vorhergehenden Ansprüche, wobei
ein Hintergrundpuffer verwendet wird, der Tris(hydroxymethyl)methyl-aminopropan-sulfonsäure unter der Bezeichnung TAPS aufweist, wenn der Chip zur Analyse von RNA- und/oder DNA-Fragmenten vorbereitet wird.

8. Verfahren nach Anspruch 1 oder einem der vorhergehenden Ansprüche, wobei der Mikrofluid-Chip eine Vielzahl über ein Fluid mit dem Mikrokanalsystem verbundener Mulden aufweist und das Verfahren ferner das Einführen eines Hintergrundpuffers in eine oder mehrere der Mulden aufweist.

9. Verfahren nach Anspruch 5, wobei
die Bedingungen für das Tiefkühlen so gewählt werden, dass eine Phasentrennung zwischen dem Hintergrundpuffer und der Gelmatrix vermieden wird.

10. Verfahren nach Anspruch 1 oder einem der vorhergehenden Ansprüche, wobei das Verfahren ferner das Schützen des Mikrofluid-Chips mittels einer Folie oder einer Abdeckung aufweist, um die Sublimation der tiefgekühlten Fluide zu verhindern.

## Revendications

1. Procédé permettant d'analyser des composés d'un échantillon (11) donné en utilisant une puce microfluidique (1) comprenant un système de microcanaux comportant au moins un microcanal (2), le procédé comprenant les étapes suivantes :
la fourniture d'un ou de plusieurs fluides à une section prédéfinie au moins du système de microcanaux ou à l'ensemble du système de microcanaux ;
la congélation (6) de la puce microfluidique (1) tout entière en exposant la puce microfluidique (1) à une température inférieure à -5□, dans laquelle le ou les fluides contenus dans le système de microcanaux sont complètement gelés ;
le transport de la puce microfluidique (7) ;
la décongélation (9) de la puce microfluidique (7) ;
la fourniture de l'échantillon (11) à un puits d'échantillons (12) ou à un microcanal dédié de la puce microfluidique (7), et
la séparation et l'analyse des composés de l'échantillon (11).

2. Procédé suivant la revendication 1, dans lequel
la puce microfluidique comprend un système de séparation d'échantillon à même de séparer, de préférence par électrophorèse, des composés d'un échantillon donné.

3. Procédé suivant la revendication 1 ou 2, dans lequel
la puce microfluidique est réalisée sous la forme d'une structure multicouche.

4. Procédé suivant la revendication 1 ou l'une quelconque des revendications précédentes, dans lequel les fluides fournis au système de microcanaux comprennent un ou plusieurs éléments du groupe comprenant un tampon de base, un tampon de base incluant une matrice de gel, un tampon d'échantillon, un tampon d'échantillon de référence, une solution de décoloration et une solution de teinture.

5. Procédé suivant la revendication 1 ou l'une quelconque des revendications précédentes, comprenant en outre : la fourniture d'un tampon de base incluant une matrice de gel à une section prédéfinie au moins du système de microcanaux ou à l'ensemble du système de microcanaux.

6. Procédé suivant la revendication 1 ou l'une quelconque des revendications précédentes, dans lequel
dans le cas où la puce est préparée en vue de son utilisation dans une analyse protéique, un tampon de base comprenant un gel Tris, Tricine et SDS est utilisé.

7. Procédé suivant la revendication 1 ou l'une quelconque des revendications précédentes, dans lequel
dans le cas où la puce est préparée pour analyser au moins un fragment d'ARN et d'ADN, un tampon de base comprenant un acide Tris(hydroxyméthyl)méthyl-aminopropane sulfonique, également appelé TAPS, est utilisé.

8. Procédé suivant la revendication 1 ou l'une quelconque des revendications précédentes, dans lequel la puce microfluidique comprend une pluralité de puits raccordés par voie fluidique au système de microcanaux, le procédé comprend en outre la fourniture d'un tampon de base à un ou plusieurs des puits.

9. Procédé suivant la revendication 5, dans lequel
les conditions de congélation sont choisies de manière à éviter la séparation de phases du tampon de base et de la matrice de gel.

10. Procédé suivant la revendication 1 ou l'une quelconque des revendications précédentes, dans lequel le procédé comprend en outre la protection de la puce microfluidique au moyen d'une feuille ou d'un cache, afin d'empêcher la sublimation des fluides congelés.
